Europäisches Patentamt

European Patent Office

Office européen des brevets

(19)

(11) Numéro de publication : **0 200 654**
**B1**

(12) **FASCICULE DE BREVET EUROPÉEN**

(45) Date de publication du fascicule du brevet :
**15.06.88**

(51) Int. Cl.⁴ : **C 07 C 11/08**, C 07 C   2/30,
    C 07 C   7/05

(21) Numéro de dépôt : **86400948.5**

(22) Date de dépôt : **30.04.86**

(54) **Procédé de production de butène-1 de pureté améliorée à partir du produit brut de dimersion de l'éthylène.**

(30) Priorité : **02.05.85 FR 8506817**

(43) Date de publication de la demande :
**10.12.86 Bulletin 86/45**

(45) Mention de la délivrance du brevet :
**15.06.88 Bulletin 88/24**

(84) Etats contractants désignés :
**BE DE GB IT NL SE**

(56) Documents cités :
**CHEMICAL ABSTRACTS, vol. 96, no. 24, 14 juin 1982,**
**page 13, résumé no. 200342g, Columbus, Ohio, US**

(73) Titulaire : **INSTITUT FRANCAIS DU PETROLE**
**4, Avenue de Bois-Préau**
**F-92502 Rueil-Malmaison (FR)**

(72) Inventeur : **Chauvin, Yves**
**64, avenue du Général Leclerc**
**F-78230 Le Pecq (FR)**
Inventeur : **Commereuc, Dominique**
**32, rue Abel Vacher**
**F-92190 Meudon (FR)**
Inventeur : **Glaize, Yves**
**Résidence Bois d'Ozon**
**F-69360 Saint-Symphorien d'Ozon (FR)**

EP 0 200 654 B1

Jouve, 18, rue St-Denis, 75001 Paris, France

## Description

L'objet de la présente invention est une production de butène-1 de pureté améliorée à partir du produit brut de dimérisation de l'éthylène. Ce procédé repose sur la découverte d'une nouvelle méthode de désactivation d'un catalyseur de dimérisation de l'éthylène formé par le mélange de titanate de tétraalkyle et de trialkylaluminium.

La dimérisation sélective de l'éthylène en butène-1 par le produit de l'interaction entre un titanate de tétraalkyl et un trialkylaluminium a été décrite pour la première fois dans le brevet des E.U. d'Amérique n° 2.943.125. D'autres brevets décrivent des améliorations apportées à ce catalyseur. Dans une réalisation industrielle, en plus de la maîtrise de la réaction elle-même se pose le problème de la séparation et de l'isolement des produits à partir du mélange brut de réaction et en particulier du catalyseur qui est totalement soluble dans le mélange réactionnel. Deux méthodes peuvent être utilisées dans ce but :

— élimination du catalyseur, avant distillation des hydrocarbures, soit par insolubilisation et filtration ou centrifugation, soit par extraction biphasique par de l'eau par exemple ;

— distillation directe des hydrocarbures (éthylène non converti, butène-1, hexènes et autres oligomères), qui laisse au fond du ballon de distillation le catalyseur, toujours soluble mais concentré dans les oligomères.

C'est cette dernière méthode qui est préférable d'un point de vue économique, car elle évite une étape intermédiaire de lavage biphasique ou de séparation de solide, et conduit à un résidu de faible volume, facile à éliminer, par exemple par incinération.

Cependant, dans la pratique, il a été observé que les températures relativement élevées qui sont atteintes lors de la vaporisation et qui sont nécessaires pour concentrer au mieux le catalyseur, provoquent des réactions secondaires d'isomérisation et de polymérisation du butène-1.

L'invention concerne ainsi un procédé de production de butène-1 de pureté améliorée à partir du produit de dimérisation de l'éthylène en présence d'un catalyseur comprenant un mélange de titanate d'alkyle et de trialkylaluminium, caractérisé en ce que l'on soumet à une distillation un mélange d'au moins une amine avec ledit produit brut de dimérisation, ladite amine répondant à la formule générale $HNR_1R_2$ dans laquelle $R_1$ et $R_2$, égaux ou différents, sont l'un l'hydrogène ou un radical hydrocarboné et l'autre un radical hydrocarboné, ou ensemble un radical alcoylène, ladite distillation ayant lieu au moins en partie à une température supérieure à la température de dimérisation, de manière à séparer au moins le butène-1 de pureté améliorée d'un fond de distillation renfermant les résidus de catalyseur. Cette valeur à partir de laquelle se produisent des réactions indésirables est habituellement d'environ 90 °C.

Les amines utilisables selon l'invention sont les amines primaires ou secondaires de formule générale $HNR_1R_2$ dans laquelle $R_1$ et $R_2$, égaux ou différents sont l'un l'hydrogène ou un radical hydrocarboné et l'autre un radical hydrocarboné. $R_1$ et $R_2$ ensemble peuvent représenter un radical alcoylène. Le radical hydrocarboné peut être aliphatique, cycloaliphatique ou aromatique et comporter par exemple, de un à vingt-deux atomes de carbone.

Pour des raisons pratiques il est souhaitable, sans être indispensable, que la tension de vapeur de l'amine soit faible, c'est-à-dire qu'elle ne vienne pas contaminer de façon appréciable les hydrocarbures lors de leur distillation et/ou leur rectification ; c'est pourquoi on utilisera de préférence les amines qui comportent en tout plus de 5 atomes de carbone, par exemple de 5 à 22, ce qui, d'autre part, favorise le maintien du catalyseur en solution. Parmi les amines on citera plus particulièrement la cyclohexylamine, l'éthyl-2 hexylamine, la laurylamine, la stéarylamine, l'oléylamine, l'aniline, la N-méthylaniline, la dibutylamine, la didécylamine, les mélanges d'amines obtenus au départ des corps gras naturels, par exemple le suif, l'huile de palme ou l'huile de coprah.

L'amine est ajoutée de préférence, comme il vient d'être dit avant distillation, de préférence à la température à laquelle la réaction de dimérisation a lieu, c'est-à-dire entre 0 et 100 °C et de préférence entre 20 et 80 °C, ou encore à une température inférieure ou légèrement plus élevée. Si la distillation débute au-dessous de 90 °C, il est encore envisageable de n'introduire l'amine qu'après le début de cette distillation, par exemple lorsque la température s'élève et dépasse, par exemple, 90 °C.

La quantité ajoutée peut varier légèrement. De préférence, elle est telle que le rapport molaire entre l'amine et le composé du titane contenu dans le mélange réactionnel soit compris entre 0,1 : 1 et 10 : 1, et de préférence de 0,3 : 1 à 2 : 1.

La distillation des hydrocarbures se fait le plus souvent entre 90 et 180 °C, sous pression atmosphérique ou sous une pression quelconque, supérieure ou inférieure, mais qui, si nécessaire, est compatible avec les autres éléments du procédé, en particulier avec le recyclage de l'éthylène, dans le cas d'un procédé en continu, et le fractionnement du butène-1.

Les exemples suivants illustrent l'invention sans en limiter la portée.

### Exemple 1

Dans un réacteur de 0,25 l muni d'un agitateur et d'une double enveloppe, on a introduit 1,47 $10^{-4}$ mole de tétrabutoxytitane et 4,5 $10^{-4}$ mole de triéthylaluminium. On maintient une pression de

0 200 654

2 MPa au moyen d'éthylène, et on régule la température à 55 °C. Quand 100 l d'éthylène ont été absorbés, on chauffe le réacteur à 140 °C pour distiller les hydrocarbures ; la teneur en butène-2 du butène-1 est, dans ces conditions de 3 500 ppm en poids. Si on répète l'opération ci-dessus mais que préalablement au chauffage à 140 °C, on introduise 10 équivalents de dodécylamine par rapport au titane, la teneur en butène-2 du butène-1 recueilli n'est que de 560 ppm en poids.

## Exemple 2

Dans un réacteur agité dont le volume total est d'environ 10 litres mais dans lequel la phase liquide n'occupe que 8,6 litres, et dont on maintient la température à 55 °C et la pression à 2,3 MPa, on introduit en continu 700 g/h d'éthylène, 0,1 g/h de triéthylaluminium et 0,1 g/h de titanate de butyle. A la sortie du réacteur, l'effluent passe dans un tube chauffé à 115 °C puis est détendu dans un volume maintenu à la même température ; le butène et l'éthylène distillent. La conversion de l'éthylène s'établit à environ 86 %. On constate que dans ces conditions la teneur moyenne en butène-2 du butène-1 est de l'ordre de 300 ppm en poids.

Lorsqu'on répète l'opération en introduisant de la décylamine dans l'effluent du réacteur avant passage dans le tube chauffé, au débit horaire de 0,035 g (rapport molaire amine/titanate introduit = 0,7) la teneur en butène-2 du butène-1 tombe à moins de 40 ppm en poids.

## Exemple 3

On opère dans les mêmes conditions que celles de l'exemple 2 à ceci près qu'on a maintenu la température du réacteur à 85 °C et la pression à 3,1 MPa. En absence d'injection d'amine, on a mesuré une teneur en butène-2 dans le butène-1 de l'ordre de 500 ppm. Quand on répète l'opération en injectant de la décylamine dans le rapport molaire amine/titanate introduit = 1, la teneur en butène-2 n'est que de 100 ppm.

## Exemples 4 à 8

On a répété l'exemple 2 en introduisant comme catalyseur 0,1 g/h de triéthylaluminium, 0,1 g/h de titanate de butyle et 0,042 g/h de tétrahydrofuranne. On a ensuite introduit diverses amines dans l'effluent du réacteur, avant passage dans le tube chauffé à 115 °C.

Les résultats sont donnés dans le tableau 1 :

Tableau 1

| Exemple | Amine | Teneur en butène-2 du butène-1 |
|---------|-------|--------------------------------|
| 4 | Piperidine (0,02 g/h) | 45 ppm |
| 5 | Cyclohexylamine (0,03 g/h) | 55 ppm |
| 6 | Ethyl-2 hexylamine (0,04 g/h) | 40 ppm |
| 7 | Oléylamine (0,08 g/h) | 50 ppm |
| 8* | Dibutylamine (0,04 g/h) | 65 ppm |

* Dans ce cas, le titanate était le titanate d'isopropyle (0,083 g/h).

**Revendications**

1. Procédé de production de butène-1 de pureté améliorée à partir du produit de dimérisation de l'éthylène en présence d'un catalyseur comprenant un mélange de titanate d'alkyle et de trialkylaluminium, caractérisé en ce que l'on soumet à une distillation un mélange d'au moins une amine avec ledit produit brut de dimérisation, ladite amine répondant à la formule générale $HNR_1R_2$ dans laquelle $R_1$ et $R_2$, égaux ou différents, sont l'un l'hydrogène ou un radical hydrocarboné et l'autre un radical hydrocarboné, ou ensemble un radical alcoylène, ladite distillation ayant lieu au moins en partie à une température supérieure à la température de dimérisation, de manière à séparer au moins le butène-1 de

3

**0 200 654**

pureté améliorée d'un fond de distillation renfermant les résidus de catalyseur.

2. Procédé selon la revendication 1 dans lequel la température de dimérisation est 0 à 100 °C et la température de distillation est de 90 à 180 °C.

3. Procédé selon la revendication 1 dans lequel la température de dimérisation est 20 à 80 °C et la température de distillation est de 90 à 180 °C.

4. Procédé selon l'une des revendications 1 à 3 dans lequel l'amine renferme de 5 à 22 atomes de carbone.

## Claims

1. A process for producing 1-butene of improved purity from the product of ethylene dimerization, in the presence of a catalyst comprising a mixture of alkyl titanate and trialkylaluminum, characterized by subjecting to distillation the mixture of at least one amine with said raw dimerization product, said amine having the general formula $HNR_1R_2$ wherein $R_1$ and $R_2$, same or different, are the one hydrogen or a hydrocarbon radical and the other one a hydrocarbon radical, or are together an alkylene radical, said distillation taking place at least in part at a temperature higher than the dimerization temperature, so as to separate at least 1-butene of improved purity from distillation bottoms comprising catalyst residues.

2. A process according to claim 1, wherein the dimerization temperature is from 0 to 100 °C and the distillation temperature from 90 to 180 °C.

3. A process according to claim 1, wherein the dimerization temperature is from 20 to 80 °C and the distillation temperature from 90 to 180 °C.

4. A process according to one of claims 1-3 wherein the amine comprises 5 to 22 carbon atoms.

## Patentansprüche

1. Verfahren zur Herstellung von Buten-1 von verbesserter Reinheit aus einem Äthylendimerisationsprodukt in Anwesenheit eines Katalysators, der eine Mischung von Alkyltitanat und Trialkylaluminium enthält, dadurch gekennzeichnet, dass man ein Gemisch von wenigstens einem Amin und dem besagten Dimerisationsrohprodukt einer Destillation unterwirft, wobei das besagte Amin der Grundformel $HNR_1R_2$ entspricht, in welcher $R_1$ und $R_2$, entweder gleich oder verschieden, der eine Wasserstoff oder ein Kohlenwasserstoffradikal und der andere ein Kohlenwasserstoffradikal, oder zusammen ein Alkylenradikal sind, wobei die besagte Destillation wenigstens teilweise bei einer Temperatur stattfindet, die höher als die Dimerisationstemperatur ist, sodass wenigstens das Buten-1 von verbesserter Reinheit von einem Destillationsboden getrennt wird, der die Katalysatorrückstände enthält.

2. Verfahren nach Anspruch 1, dadurch gekennzeichnet, dass es eine Dimerisationstemperatur von 0 bis 100 °C und eine Destillationstemperatur von 90 bis 180 °C beträgt.

3. Verfahren nach Anspruch 1, dadurch gekennzeichnet, dass es eine Dimerisationstemperatur von 20 bis 80 °C und eine Destillationstemperatur von 90 bis 180 °C beträgt.

4. Verfahren nach einem der Ansprüche 1 bis 3, dadurch gekennzeichnet, dass das Amin 5 bis 22 Kohlenstoffatome enthält.

4